# EUROPEAN PATENT SPECIFICATION

(11) **EP 1 707 140 B1**
(45) Date of publication and mention of the grant of the patent: **14.09.2011**
(21) Application number: 06251814.7
(22) Date of filing: 31.03.2006
(51) Int. Cl.: A61B 17/80

(54) **Navicular fixation device**
Fixationsvorrichtung für das Fusskahnbein
Dispositf de fixation pour l'os naviculaire

(30) Priority: 31.03.2005 US 96084
(43) Date of publication of application: 04.10.2006
(73) Proprietor: DePuy Products, Inc., Warsaw, IN 46581 (US)
(72) Inventor: Sanders, Roy, Tampa (US); Prasad, Priya, Warsaw IN 46580 (US); Bremer, Chris, Warsaw IN 46582 (US)
(74) Representative: Gover, Richard Paul

(56) References cited:
- DE-U- 20 300 181
- FR-A- 2 622 431
- US-A- 6 123 709
- US-B1- 6 348 052
- SCHILDHAUER T A ET AL: "Temporary bridge plating of the medical column in severe midfoot injuries" JOURNAL OF ORTHOPAEDIC TRAUMA 2003 UNITED STATES, vol. 17, no. 7, 2003, pages 513-520, XP008066134 ISSN: 0890-5339
- KHAN K M ET AL: "Trasal navicular stress fracture in athletes" SPORTS MEDICINE 1994 NEW ZEALAND, vol. 17, no. 1, 1994, pages 65-76, XP008066198 ISSN: 0112-1642

## Description

The present invention relates to fixation devices for the bones of the foot, and particularly for the navicular bone. The invention is especially suitable for reducing comminuted fractures of the navicular.

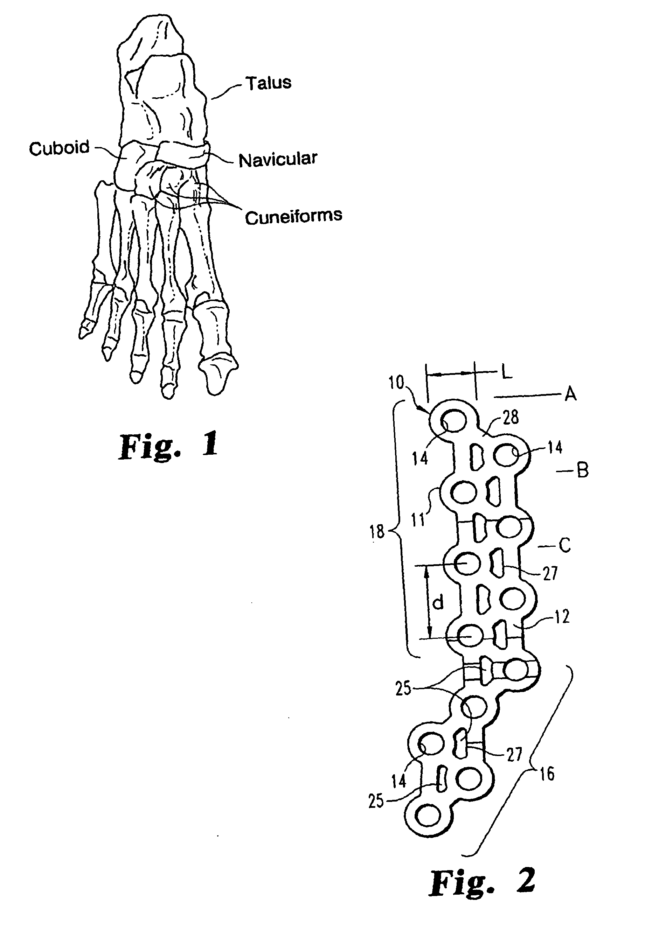

The foot is formed by a complex array of bones and ligaments that all present unique signatures when injured. The navicular bone is situated between the talus and the cuneiform bones and medially adjacent the cuboid, as shown in FIG. 1. The navicular serves as an anchor for medial or internal ligaments of the ankle, as well as for ligaments connected to the cuboid and metatarsal bones. The navicular is particularly susceptible to stress fractures in physically active persons. For instance, during foot strike, the navicular bone is impinged between the proximal talus and the distal cuneiforms, with most of the force focused at the central one-third of the bone. The risk of stress fracture of the navicular is aggravated by the fact that this high stress region of the bone is an area of relative avascularity.

Physical activities involving repetitive foot strikes, especially those involving antagonistic muscle load, are thought to eventually result in bone failure. The onset of navicular stress fractures are often difficult to diagnose. These fractures are usually signaled by a vague aching pain in the dorsal mid-foot that may radiate along the medial arch. The pain typically increases with activity such as running and jumping, and is frequently masked by the patient by altering the gait and decreasing the use of the forefoot. The pain usually resolves rapidly with rest. However, until a positive diagnosis is made, repeated physical activity results in increased stress and bony resorption focused at the central one-third of the bone, eventually resulting in a vertical fracture of the navicular.

In many cases, navicular stress fractures may be treated simply by immobilization of the ankle using a non-weight bearing cast. However, in some cases, the conservative treatment fails to achieve union of the fracture. More severe injuries involving a displaced fracture or fragmentation cannot be treated by the conservative methods and require surgical intervention. In the past, the primary surgical approach has been to implant bone screws or pins across the fracture, sometimes augmented with a bone graft inlay. Where the fracture is fragmented, multiple screws/pins are required, which significantly complicates the procedure and increases the risk of non-union. In the case of severely comminuted fractures, screws/pins are inadequate to align and reduce the bone fragments.

Schildhauer T.A. et al, "Temporary bridge plating of the medial column in severe midfoot injuries", J. Orthopedic Trauma, vol. 17, no. 7, 2003, pp. 513-520, discloses a plate attachable to the medial amongst other bones.

DE-U-20 300 181 discloses elongated bone fixation plates in which portions defining a plurality of screwholes along the length of the plate are interconnected by scouts defining a plurality of cut-outs through the plate.

What is needed is a fixation device that is specifically adapted for the navicular bone. The fixation device must be capable of reducing fractures of the navicular bone, from the simple vertical fracture to the more problematic comminuted fracture.

Embodiments of the present invention address this need by an elongated plate defining a plurality of holes along the length of said plate, the holes being configured to receive a bone screw. The plate includes a medial section configured for attachment to the medial aspect of the navicular and a dorsal section that forms a hook shape for attachment to the dorsal aspect of the navicular.

According to the present invention there is provided a fixation device for the navicular bone of the foot, comprising: an elongate plate defining a plurality of holes along the length of said plate, said holes being configured to receive bone screws through them, the plate including a medial section configured for attachment to the medial aspect of the navicular and a dorsal section; wherein said plate comprises struts interconnecting the portions of said plate defining each adjacent hole, said struts defining a plurality of cut-outs through said plate; characterised in that said dorsal section is formable into a hook shape or is formed as a hook shape for attachment to the dorsal aspect of the navicular and said plurality of cut-outs are configured to receive smaller bone screws through them such that the bone screw head lodges at the corners of the cut-outs to secure the fixation device to the navicular.

In one embodiment of the invention, the plurality of holes includes a first number of holes defined in the medial section and a second number of holes in the dorsal section that is greater than or equal to the first number of holes. In a specific embodiment, four holes are provided in the medial section and 4, 6 or eight holes are defined in the dorsal section, depending upon the size of the fixation plate being used for the particular patient. In the preferred embodiment, the medial section and the dorsal section are offset relative to each other transverse to the length of said plate to better conform to the anatomy of the navicular bone.

In a further embodiment, the plurality of holes are in spaced, staggered arrangement relative to each other in which each hole is offset along the length of the plate and transverse to the length of said plate relative to any adjacent hole. In the preferred embodiment, the plurality of holes are offset along the length of said plate by a substantially equal offset dimension, which may be between about 5.8 mm and 7.5 mm. Adjacent ones of the plurality of holes may be offset transverse to the length of the plate by between about 5.6 mm and 6.4 mm.

In certain embodiments, at least some of the plurality of holes include a circumferential chamfer so that the holes are configured to receive differently sized fasteners. In a specific embodiment, the chamfered screw holes accept 2.7 mm or 3.5 mm screws.

In the invention, the plate includes a strut interconnecting the portion of the plate defining each adjacent hole. The struts then define a plurality of cut-outs through the plate. The cut-outs are sized and configured to receive additional bone engaging fasteners, thereby providing additional points for fixation and reduction that are especially useful for severely comminuted fractures.

In the preferred embodiment, the hook shape of the dorsal section includes a first portion adjacent the medial section that is bent at a first radius and a second portion bent at a second radius less than the first radius. The first section helps provide a transition to the hook shape, while the hook shaped second section is useful in holding the fragments of the navicular bone together.

It is the object of the invention to provide a device for fixation of the navicular bone of the foot. One benefit of the invention is that the fixation device may be used for simple vertical fractures of the navicular or more problematic comminuted fractures. A further benefit is that the plate presents a minimal prominence when attached to the navicular bone.

Embodiments of the invention will now be described by way of example with reference to the accompanying drawings, in which:
FIG. 1 is a view of the dorsal aspect of the bones of a right foot.
FIG. 2 is a plan view of a navicular fixation device in accordance with one embodiment of the invention.
FIG. 3 is a side view of the fixation device shown in FIG. 2 formed into its final configuration for fixation of the navicular.
FIG. 4 is a lateral view of the fixation device shown in FIG. 3.
FIG. 5 is a side view of the bones of a left foot with the device shown in FIGS. 3-4 positioned on the navicular bone.

Referring to the drawings, FIGS. 2 to 4 show a navicular fixation device 10 in the form of a truss plate 11. The plate is formed of a medical grade material, such as a biocompatible metal. In the preferred embodiment, the metal is a titanium alloy, such as Ti-6A1-4V. Since the truss plate 11 is intended to be a permanent implant, it is desirable to minimize the prominence of the plate above the bone. Thus, in one feature of the invention, the plate has a thickness of about 0.9 to 1.0 mm. This thickness allows the plate to have sufficient strength and stiffness, while maintaining a very low profile on the navicular. Moreover, this thickness allows the truss plate 11 to be formed *in situ* as necessary to fit the specific anatomy of the bone.

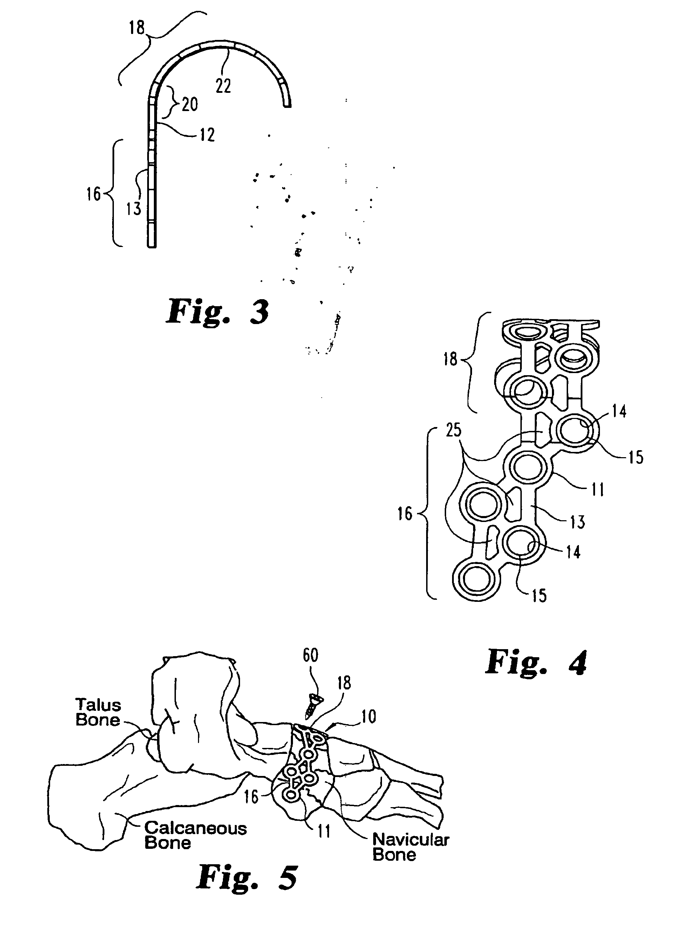

The truss plate 11 includes a plurality of holes 14 configured to receive a bone engaging fastener. In embodiments according to the invention, the fastener is a bone screw, such as the screw 60 depicted in FIG. 5. The screw is sized to fit within the navicular bone, and especially to engage bone segments in the event of a comminuted fracture. As shown in FIG. 2, two adjacent rows of screw holes extend along the length of the plate. The screw holes 14 are arranged in an alternating staggered pattern in order to ensure that bone screws passing through adjacent holes 14 do not interfere with each other. In a specific embodiment, the screw holes are spaced at about 5.8 mm intervals d along the length of the plate and about 6.35 mm apart across the width of the plate. In addition, the alternating pattern minimizes the overall width of the adjacent rows. In the embodiment shown in FIG. 2, the adjacent rows are offset along the width of the plate by about 6.35 mm. In another specific embodiment, the rows are spaced about 5.6 mm apart.

In a further feature of the plate 11, the screw holes are arranged into a medial section 16 and a dorsal section 18. The medial section 16 includes a pattern of four screw holes that are configured for attachment to the medial aspect of the navicular, as shown in FIG. 5. The dorsal section 18 includes a pattern including a variable number of screw holes depending upon the size of the plate, as explained in more detail below. This section is configured to span the dorsal aspect of the bone, as shown in FIG. 5, and as explained below. In order to best accommodate the anatomy of the navicular bone, the medial section 16 is offset from the dorsal aspect 18 by a dimension L. This offset allows the plate 11 to conform to the lateral curvature of the navicular. In a specific embodiment, the offset L is about 6.35 mm for a large or medium size plate and about 5.59 mm for a small plate. In addition to the lateral offset, the screw holes 14 in the medial section 16 are generally aligned along an axis that is at a slight angle relative to the axis of alignment of the screws of the dorsal section 18.

Although the plate 11 is shown generally flat in FIG. 2, in the preferred embodiment, the dorsal section 18 of the plate is curved to generally form a "hook" profile, as depicted in FIG. 3. In the most preferred embodiment, the section incorporates two portions with different curvatures, in which the first section 20 has a more shallow curvature than the second section 22. In a specific embodiment, the first section 20 is formed at a radius of about 50.8 mm, while the "hook" shaped second section 22 is bent at a radius of about 12.7 mm. This hook configuration helps the plate 11 conform more closely to the natural anatomy of the navicular, but perhaps more importantly, the hook shape aids in reduction of fractures of the navicular. In particular, when the plate 11 is implanted onto the navicular, as shown in FIG. 5, the dorsal section 18 fits snugly over the top of the bone, drawing any bone fragments together into their natural anatomic position to fully reform the navicular bone.

In one aspect of the preferred embodiment, the screw holes 14 are provided with a circumferential chamfer 15, as shown in FIG. 4. The chamfer allows the holes to accommodate differently sized screws. In the most preferred embodiment, the screw holes 14 and chamfer 15 are configured to receive 2.7 mm or 3.5 mm screws. Thus, the surgeon can select either size screw to be introduced through any of the plurality of holes 14 as is best suited for the particular fracture and bone anatomy.

It can also be appreciated that the plurality of holes 14 allow the surgeon to select the proper combination of holes for attachment of the plate to the bone, and for fixation and reduction of the bone fracture. To that end, the present invention contemplates that differently sized truss plates 11 may be provided for differently sized navicular bones. Thus, as shown in FIG. 2, the plate 11 is a large plate, with a longitudinal length indicated by the line A. In a specific embodiment, the length A is about 63.4 mm and is sized to the navicular bone of a large patient. As shown in FIG. 2, the large plate includes four pairs of screw holes spanning the dorsal section 18. The intermediate length, designated by the line B, is about 51.9 mm and eliminates the last of the pair of screws holes. The smallest plate is formed by eliminating the last two pairs of screw holes above the line C in FIG. 2, and has a length of about 52.5 mm. In this shorter plate, it is contemplated that the spacing of screw holes along the plate length may be modified from the spacing for the other two plates having more pairs of screw holes in the dorsal section. Thus, in a specific embodiment for the smallest plate, the spacing interval d for the screw holes may be about 7.5 mm. Thus, although the plate defined at the line C in FIG. 2 may be longer than the intermediate plate, it includes fewer screw holes since the smaller navicular is not able to accept the larger number of screws that can be implanted with the larger two plate configurations.

The truss plate 11 is configured to minimize the amount of material while providing sufficient strength to reduce most fractures of the navicular and hold that reduction until union occurs. Thus, in a further feature of the invention, the plate includes a plurality of cut-outs 25 dispersed between each screw hole location. The cut-outs 25 are essentially defined by a series of struts, including long struts 25 that interconnect longitudinally spaced screw hole locations along the length of the plate, and short struts 28 that laterally interconnect the staggered hole locations. The short struts 28 are nominally oriented at an angle relative to the longitudinal axis of the plate so that the cut-outs 25 are generally rhomboid.

While the cut-outs 25 and struts 27, 28 reduce the overall material of the plate 11, they also serve other valuable functions. One benefit is that the struts allow the plate to be bent as needed to more accurately fit the complex geometry of the navicular. Another important benefit is that the cut-outs 25 provide additional bone fastener positions. These additional positions may be especially important for fixation and reduction of a severely comminuted navicular fracture. The cut-outs 25 are sized so that pins may be directed through them to help reform the comminuted fractures. Smaller bone screws may also extend through the cut-outs, with the screw head lodging at the corners of the cut-outs 25. In the embodiment illustrated in FIG. 2, the screw holes 14 and cut-outs 25 provide twenty-one locations for a bone fastener or bone screw. Even in the smaller plate that includes eight screw holes, the cut-outs add five additional fixation/reduction locations.

As shown in FIG. 5, and as explained briefly above, the truss plate 11 includes curved portions 20, 22 that fit over the dorsal aspect of the navicular. To implant the device 10, the navicular is accessed dorsally and the plate is directed over the bone, with the medial section 16 following the medial aspect of the bone. As the plate fits over the navicular, it helps draw the bone segments together. In the typical case, the navicular fracture is vertical, so the hook shape of the plate 11 is particularly well-suited to reduce the fracture. Even for severely comminuted fractures, the hook shape helps collect and reduce the fragments. In some cases, the plate may require minor contouring to match the geometry of the navicular. However, in the typical case, the plate may be simply positioned over the navicular and attached to the bone using appropriately sized bone screws. In a preferred procedure, the dorsal section 18 may be fastened first, with particular attention to restoring bone fragments and reducing all fractures of the bone. The surgeon can select a wide variety of screw arrangements as dictated by the fractures. Once the dorsal section has been attached to the bone, the medial section 16 may then be attached with a desired number of bone fasteners.

## Claims

1. A fixation device (10) for the navicular bone of the foot, comprising:
an elongate plate (11) defining a plurality of holes (14) along the length of said plate (11), said holes (14) being configured to receive bone screws (60) through them, the plate (11) including a medial section (16) configured for attachment to the medial aspect of the navicular and a dorsal section (18);
wherein said plate (11) comprises struts (27, 28) interconnecting the portions of said plate (11) defining each adjacent hole (14), said struts (27, 28) defining a plurality of cut-outs (25) through said plate;
**characterised in that** said dorsal section (18) is formable into a hook shape or is formed as a hook shape for attachment to the dorsal aspect of the navicular and said plurality of cut-outs (25) are configured to receive smaller bone screws through them such that the bone screw head lodges at the corners of the cut-outs (25) to secure the fixation device (10) to the navicular.

2. The fixation device (10) of claim 1, wherein said plurality of holes (14) includes a first number of holes (14) defined in said medial section (16) and a second number of holes (14) in said dorsal section (18) that is greater than or equal to said first number of holes (16).

3. The fixation device (10) of claim 2, wherein said medial section (16) and said dorsal section (18) are offset relative to each other transverse to the length of said plate (11).

4. The fixation device (10) of claim 1, wherein said plurality of holes (14) are in spaced, staggered arrangement relative to each other in which each hole (14) is offset along the length of said plate (11) and transverse to the length of said plate (11) relative to any adjacent hole (14).

5. The fixation device (10) of claim 4, wherein said plurality of holes (14) are offset along the length of said plate (11) by a substantially equal offset dimension.

6. The fixation device (10) of claim 5, wherein said offset dimension is between about 5.8 mm and 7.5 mm.

7. The fixation device (10) of claim 4, wherein adjacent ones of said plurality of holes (14) are offset transverse to the length of said plate by between about 5.6 mm and 6.4 mm.

8. The fixation device (10) of claim 1, wherein at least some of said plurality of holes (14) includes a circumferential chamfer (15) to accept differently sized fasteners (60) through them.

9. The fixation device (10) of claim 1, wherein when the fixation device (10) is formed as a hook shape said dorsal section (18) includes a first portion (20) adjacent said medial section (16) that is bent at a first radius and a second portion (22) bent at a second radius less than said first radius to form said hook shape.

10. The fixation device (10) of claim 1, wherein said plate (11) has a thickness of not more than about 1.0 mm.

## Patentansprüche

1. Fixiervorrichtung (10) für den Kahnbeinknochen des Fußes, die aufweist:
eine längliche Platte (11), welche eine Vielzahl von Löchern (14) über die Länge der Platte (11) definiert, wobei die Löcher (14) so ausgelegt sind, dass sie Knochenschrauben (60) hindurch aufnehmen, wobei die Platte (11) einen mittigen Abschnitt (16), der für die Anbringung an dem mittigen Aspekt des Kahnbeins ausgelegt ist, und einen Rückenabschnitt (18) umfasst;
wobei die Platte (11) Streben (27, 28) aufweist, welche die Bereiche der Platte (11) miteinander verbinden, die jedes benachbarte Loch (14) definieren, wobei die Streben (27, 28) eine Vielzahl von Ausschnitten (25) durch die Platte definieren;
**dadurch gekennzeichnet, dass** der Rückenabschnitt (18) in eine Hakenform formbar ist oder als eine Hakenform gebildet ist, zum Anbringen an dem dorsalen Aspekt des Kahnbeins, und die Vielzahl der Ausschnitte (25) so ausgelegt ist, dass sie kleinere Knochenschrauben hindurch aufnehmen, so dass der Knochenschraubenkopf an den Ecken der Ausschnitte (25) liegt, um die Fixiervorrichtung (10) an dem Kahnbein zu sichern.

2. Fixiervorrichtung (10) nach Anspruch 1, bei der die Vielzahl der Löcher (14) eine erste Anzahl von Löchern (14), die in dem mittigen Abschnitt (16) definiert ist, und eine zweite Anzahl von Löchern (14) in dem Rückabschnitt (18), die größer als die oder gleich der ersten Anzahl von Löchern (16) ist, umfasst

3. Fixiervorrichtung (10) nach Anspruch 2, bei der der mittige Abschnitt (16) und der Rückenabschnitt (18) in Bezug aufeinander quer zu der Länge der Platte (11) versetzt sind.

4. Fixiervorrichtung (10) nach Anspruch 1, bei der die Vielzahl der Löcher in Bezug aufeinander beabstandet und abgestuft angeordnet ist, wobei jedes Loch (14) entlang der Länge der Platte (11) und quer zu der Länge der Platte (11) in Bezug auf irgendein benachbartes Loch (14) versetzt ist.

5. Fixiervorrichtung (10) nach Anspruch 4, bei der die Vielzahl der Löcher (14) über die Länge der Platte (11) über einen im Wesentlichen gleichen Versatzbetrag versetzt sind.

6. Fixiervorrichtung (10) nach Anspruch 1, bei der der Versatzbetrag zwischen etwa 5.8 mm und 7.5 mm liegt.

7. Fixiervorrichtung (10) nach Anspruch 4, bei der benachbarte aus der Vielzahl der Löcher (14) quer zu der Länge der Platte um zwischen etwa 5.6 mm und 6.4 mm versetzt sind.

8. Fixiervorrichtung (10) nach Anspruch 1, bei der wenigstens einige aus der Vielzahl der Löcher (14) eine Umfangsschräge (15) umfassen, um unterschiedlich bemessene Befestigungselemente (16) aufzunehmen.

9. Fixiervorrichtung (10) nach Anspruch 1, bei der, wenn die Fixiervorrichtung (10) als eine Hakenform gebildet ist, der Rückabschnitt (18) einen ersten Bereich (20) benachbart dem mittigen Abschnitt (16) umfasst, der mit einem ersten Radius gebogen ist, und einen zweiten Abschnitt (22), der mit einem zweiten Radius gebogen ist, welcher kleiner ist als der erste Radius, um die Hakenform zu bilden.

10. Fixiervorrichtung (10) nach Anspruch 1, bei der die Platte (11) eine Dicke von nicht mehr als etwa 1.0 mm hat.

## Revendications

1. Dispositif de fixation (10) destiné à l'os naviculaire du pied, comprenant :
une plaque allongée (11) qui définit une pluralité de trous (14) sur la longueur de ladite plaque (11), lesdits trous (14) étant configurés de façon à recevoir des vis à os (60) à travers ceux-ci, la plaque (11) comprenant une section médiale (16) configurée pour une fixation à l'aspect médial de l'os naviculaire et une section dorsale (18) ;
dans lequel ladite plaque (11) comprend des entretoises (27, 28) qui relient ensemble les parties de ladite plaque (11) qui définissent chaque trou adjacent (14), lesdites entretoises (27, 28) définissant une pluralité de découpes (25) à travers ladite plaque ;
**caractérisé en ce que** ladite section dorsale (18) peut être formée en une forme de crochet ou est formée en une forme de crochet pour une fixation à l'aspect dorsal de l'os naviculaire et ladite pluralité de découpes (25) sont configurées de façon à recevoir de plus petites vis à os à travers celles-ci de telle sorte que la tête de la vis à os se loge au niveau des coins des découpes (25) de façon à fixer le dispositif de fixation (10) à l'os naviculaire.

2. Dispositif de fixation (10) selon la revendication 1, dans lequel ladite pluralité de trous (14) comprend un premier nombre de trous (14) définis dans ladite section médiale (16) et un second nombre de trous (14) dans ladite section dorsale (18) qui est supérieur ou égal audit premier nombre de trous (16).

3. Dispositif de fixation (10) selon la revendication 2, dans lequel ladite section médiale (16) et ladite section dorsale (18) sont décalées l'une par rapport à l'autre de manière transversale par rapport à la longueur de ladite plaque (11).

4. Dispositif de fixation (10) selon la revendication 1, dans lequel ladite pluralité de trous (14) sont dans un agencement où ils sont espacés et décalés les uns par rapport aux autres, dans lequel chaque trou (14) est décalé sur la longueur de ladite plaque (11) et de manière transversale par rapport à la longueur de ladite plaque (11) par rapport à n'importe quel trou adjacent (14).

5. Dispositif de fixation (10) selon la revendication 4, dans lequel ladite pluralité de trous (14) sont décalés sur la longueur de ladite plaque (11) d'une dimension de décalage sensiblement égale.

6. Dispositif de fixation (10) selon la revendication 5, dans lequel ladite dimension de décalage est comprise entre environ 5,8 mm et 7,5 mm.

7. Dispositif de fixation (10) selon la revendication 4, dans lequel les trous adjacents de ladite pluralité de trous (14) sont décalés de manière transversale par rapport à la longueur de ladite plaque d'une dimension comprise entre environ 5,6 mm et 6,4 mm.

8. Dispositif de fixation (10) selon la revendication 1, dans lequel certains au moins de ladite pluralité de trous (14) comprennent un chanfrein circonférentiel (15) de façon à accepter des attaches de dimensions différentes (60) à travers ceux-ci.

9. Dispositif de fixation (10) selon la revendication 1, dans lequel, lorsque le dispositif de fixation (10) est formé en une forme de crochet, ladite section dorsale (18) comprend une première partie (20) adjacente à ladite section médiale (16) qui est pliée selon un premier rayon et une seconde partie (22) pliée selon un second rayon inférieur audit premier rayon de façon à former ladite forme de crochet.

10. Dispositif de fixation (10) selon la revendication 1, dans lequel ladite plaque (11) présente une épaisseur qui n'est pas supérieure à environ 1,0 mm.
